# EUROPEAN PATENT SPECIFICATION

(11) **EP 4 418 854 B1**
(45) Date of publication and mention of the grant of the patent: **16.04.2025**
(21) Application number: 23798298.8
(22) Date of filing: 19.10.2023
(51) Int. Cl.: A01K 43/08, G01N 33/08

(54) **METHOD AND SYSTEM FOR PROCESSING A PLURALITY OF AVIAN EGGS**
VERFAHREN UND SYSTEM ZUR VERARBEITUNG MEHRERER VOGELEIERN
PROCÉDÉ ET SYSTÈME DE TRAITEMENT D'UNE PLURALITÉ DOEUFS AVIAIRES

(30) Priority: 19.10.2022 NL 2033355
(43) Date of publication of application: 28.08.2024
(73) Proprietor: Moba Group B.V., 3771 VE Barneveld (NL)
(72) Inventor: PELLARIN, Lars, 3771 VE Barneveld (NL); DABROS, Mads Hartmann, 3771 VE Barneveld (NL)
(74) Representative: V.O.
(86) International application number: PCT/NL2023/050548
(87) International publication number: WO 2024/085757

(56) References cited:
- EP-A1- 3 939 421
- WO-A1-2010/143953
- CN-A- 1 934 935
- NYALALA INNOCENT ET AL: "Weight and volume estimation of poultry and products based on computer vision systems: a review", POULTRY SCIENCE, vol. 100, no. 5, 1 May 2021 (2021-05-01), Oxford, pages 101072, XP093037905, ISSN: 0032-5791, DOI: 10.1016/j.psj.2021.101072

## Description

The invention relates to a method and system for processing a plurality of avian eggs, for example poultry eggs, in particular unfertilized eggs.

Egg detection systems are known and marketed by the applicant. An example is the MOBA egg inspector (see www.moba.com), which includes camera's and special lighting, as well as software, for detecting leaking- and dirty eggs on the infeed of egg graders.

Further, WO2010074572 discloses a method for classifying eggs (the eggs each possessing different parts, namely an egg shell, egg fluid and an air cell), the method comprising: candling each egg to obtain at least one image of the egg, such that the different egg parts are distinguishable in the image, and processing the at least one image to classify the egg. The volume of the egg fluid of the egg may be determined by processing the at least one image. According to a further elaboration the known method, the at least one image is processed utilizing reference values, such that therewith the masses of each of the egg parts of the egg are determined. According to an extra advantageous elaboration, the mass of the egg is determined by processing the at least one image by determining the masses of each of the egg parts (for example, by determining the mass of the shell as well as the mass of the egg fluid, and utilizing associated reference values).

WO 2010/143953 discloses a further method of image based weight classification of eggs.

The present invention aims to provide an improved method for processing a plurality of eggs, in particular for classifying the eggs. An aim of the invention is to provide a method that can determine the mass of the eggs accurately and efficiently, in a reliable, economical manner, in particular such that a large number of eggs can be processed in a relatively short processing period.

According to an aspect of the invention this is achieved by the features of claim 1.

The present disclosure provides a method for processing a plurality of avian eggs, in particular unfertilized eggs, including:
- conveying each egg along a conveying path;
- generating a plurality of images of each egg from different sides of the egg using illumination light; and
processing the plurality of images of each egg, by a digital image processor, utilizing a trained learning model to determine a mass of the egg, and outputting the determined mass of the egg, for example for classifying the egg.

It has been found that in this way, surprisingly good and reliable egg mass estimations can be achieved, in case of processing a relatively large number of eggs. Determined egg masses can e.g. be subsequently used for grading/sorting the eggs, and for packaging the egg (e.g. in cartons or on trays) in a packing process. In particular, the processing does not have to determine masses of individual egg sections (such a shell mass and an egg liquid mass). The processing can only use relatively unprocessed images of the eggs (e.g. raw image data, or partly processed image data, the image data for each egg preferably including the entire contour of that egg) to be fed into a trained learning model, to provide a good egg mass determination of each of the eggs. Besides, in this way, efficient image processing can be achieved.

Good results can be achieved in case the learning model has been trained through machine learning with training data concerning a plurality of (learning model training) eggs, the training data including a plurality of images of each of the plurality of (learning model training) eggs and determined masses of the (learning model training) eggs. According to a preferred embodiment, the trained learning model includes a neural network, preferably a CNN (Convolutional Neural Network). It has been found that in this way, optimum data processing can be achieved, real-time, to provide sufficient reliable egg mass detection results, allowing carrying out the processing by relatively low power (and from economical viewpoint relatively inexpensive) image processing means. In particular, it has been found that egg masses can be determined with high accuracy, in particular providing a low standard deviation of 1 gram or even better.

It is preferred that a relatively large number of images is taken from each egg, to be used in the processing, wherein the images are preferably from mutually different viewing angles (i.e. each image is taken from another angle with respect to an egg's outer surface), which has been found to significantly contribute to improved mass estimation accuracy. For example the plurality of images can include at least 4 images of the egg and preferably at least 10 or at least 20 images, for example 25 images or more, for example in the range of 20-30 images. Preferably, the set (i.e. plurality) of images of each egg covers/shows the entire outer surface of that egg. The plurality of images of each egg can be generated by a single camera, but preferably, at least two (mutually spaced-apart) cameras are used for imaging each egg. The trained learning model can e.g. use substantially raw (i.e. substantially unprocessed) image data provided by each camera, or partly processed image data.

An aspect of the present invention provides a system for processing a plurality of avian eggs, in particular unfertilized eggs. The system includes:
- a conveyor for conveying each egg along a conveying path;
- an imaging system having at least one camera and at least one light source, for generating a plurality of images of each egg conveyed by the conveyor, from different sides of the egg, wherein the at least one camera and the at least one light source are arranged on opposite sides of the conveying path;
- a digital image processor configured to receive the plurality of images from the imaging system, and to process the plurality of images of each egg utilizing a trained learning model to determine a mass of the egg, wherein the image processor is configured to output a determined mass of the egg, for example for generating and/or storing the determined mass in association with a predetermined egg identifier of the respective egg and/or for classifying the eggs.

In this way, the above-mentioned advantages can be achieved.

Further, the invention provides a digital image processor comprising means for carrying out the processing of the plurality of images of each egg, utilizing the trained learning model to determine a mass of the egg, of the method according to the invention.

In addition, an aspect of the invention provides a method of training a machine learning model to predict the mass of an avian egg, including:
- providing a machine learning model, which is adapted to receive input data and to output prediction data, wherein the training includes (in arbitrary order):
   - A) a conveyor conveying a plurality of eggs along a conveying path;
   - B) an imaging system generating a plurality of training images of each of the eggs (being conveyed by the conveyor) from different sides of the egg, using illumination light;
   - C) measuring the (actual) mass of each of the eggs, for example by a dedicated egg mass measuring device; and
   - D) feeding the plurality of training images of each of the eggs and the measured mass of that egg to the machine learning model to train the learning model to output egg mass as prediction data.

Preferably, sets of training images and corresponding measured masses of at least 10,000 different eggs are fed to the machine learning model to train the learning model, which has been found to provide to good, reliable training data results. For example, sets of training images and corresponding measured of at least 50,000 different eggs can be fed to the machine learning model to train the learning model which provides a learned model that can achieve reliable and accurate egg mass determination (during subsequent machine operation).

Further extra advantageous embodiments of the invention are provided in the dependent claims.

The invention will now be explained in more detail, with reference to the drawing. Therein shows:
Figure 1 schematically part of a non-limiting example of an egg processing system, in a top view, according to an embodiment of the invention;
Figure 2 a cross-section over line II-II of Fig. 1;
Figure 3 schematically depicts an example of image processing of an embodiment of the invention;
Figure 4 schematically depicts a detail Q of Figure 3; and
Figure 5 shows a graph of processing results of an embodiment of the invention.

Similar or corresponding features are denoted by similar or corresponding reference signs in this application.

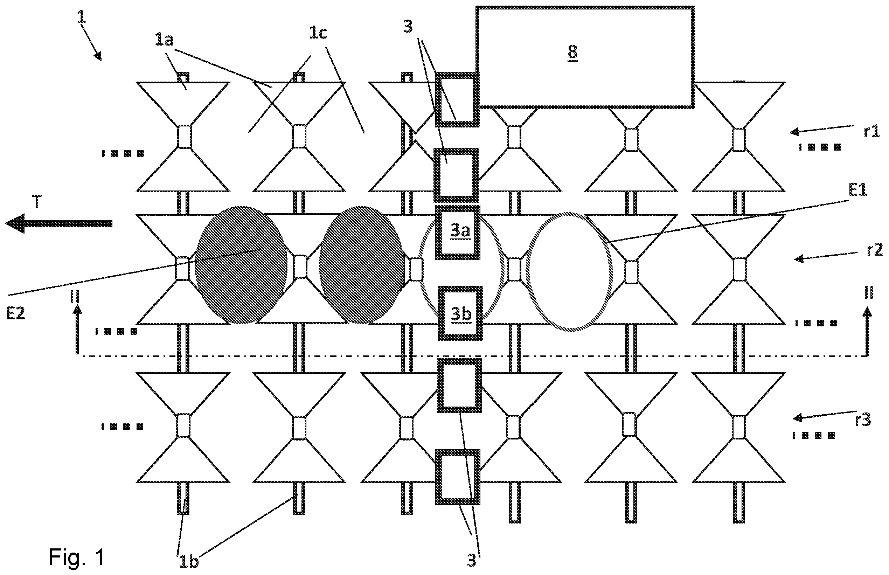

Figures 1-2 depict an egg processing system, including a conveyor 1 (only part being shown), configured for conveying a plurality of eggs E1, E2 along a conveying path (in a transport direction T), in particular as a number of rows of eggs E1, E2. Three parallel rows r1, r2, r3 are depicted in this example; naturally, the conveyor 1 may be configured to convey the eggs in more or less than three rows.

As is shown in the drawing, during use, the eggs can have different egg shell colors. For example, the drawing shows each first egg E1 having a first eggshell color that is white, wherein eggshell color of each second egg E2 is nonwhite, for example brown. Optionally, the present system can examine both types of eggs E1, E2 in a straight-forward manner.

Preferably, the conveyor 1 is an endless conveyor, e.g. an endless roller conveyer 1. It can be configured for rotating/turning the eggs during transport, e.g. around respective longitudinal egg axes. In particular, the roller conveyor can include egg supporting components, for example parallel diabolo shaped (preferably rotating) rollers 1a, defining nests there-between for receiving (and rotating) the eggs E1, E2. The egg supporting elements (e.g. rollers) 1a can be mounted on respective shafts 1b that can be driven by suitable drive means (e.g. a motor, transmission belts or chains and the-like, not shown) for moving the shafts and rollers in the conveying direction T.

It follows that the conveyor 1 can include or define egg receiving nests 1c that are partly open at respective lower (egg supporting) sides, allowing light transmission along respective egg supporting elements (in this case along rollers) 1a.

The system further includes an imaging system 2, 3, which has at least one light source 2 and at least one camera 3, for taking a plurality (N) of images of each of the eggs E.

At least one or each light source 2 can be configured for emitting a respective illumination beam B, for example of near infrared (NIR) light, towards the egg conveying path, for illuminating the eggs E1, E2 during operation. Alternatively, at least one or each light source 2 can be configured for emitting white light (which can e.g. be reflected by the egg), or blue light (which can in particularly be advantageous in case of imaging air chambers of white eggs).

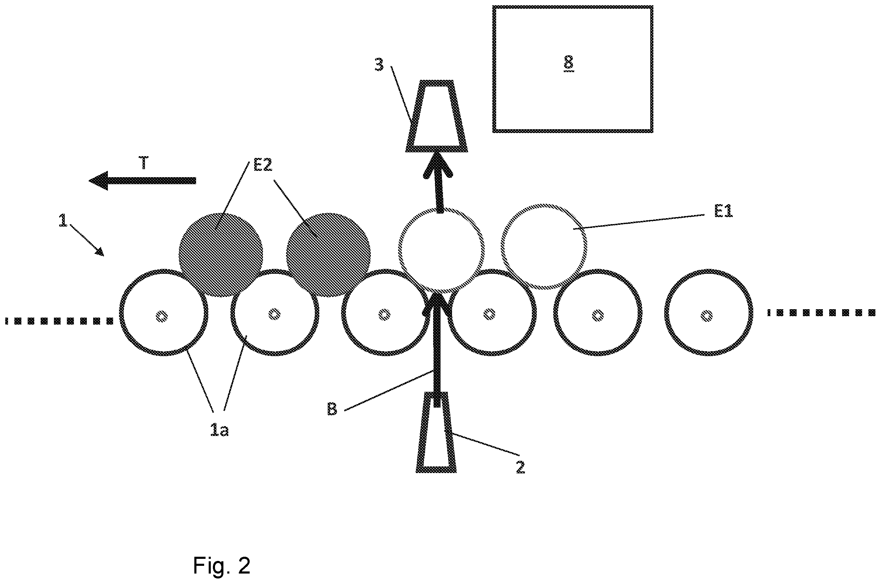

Each light beam source 2 (one being depicted) can be arranged at a vertical level below a vertical level of the egg conveying path (see Fig. 2), but that is not required. The present light source 2 is arranged to emit the light beam upwardly, so that passing eggs E1, E2 of a respective row r2 are subsequently illuminated by the beam B (the beam entering respective egg receiving nests via respective open sides of those nests, in this example).

Alternatively, a light source 2 can be arranged to emit the light beam downwardly or sideways, for illuminating passing eggs E1, E2 of a respective row r2 with the respective light beam B.

The system can include various numbers of light sources 2, wherein each source 2 can be configured to emit one or more beams B e.g. for illuminating passing eggs of one or more conveyor rows r1, r2, r3. In a non-limiting example, the light source includes one or more light emitting diodes (LEDs) for emitting the beam B.

The system can include various numbers of light sources 2, wherein each source 2 can be configured to emit one or more beams B e.g. for illuminating passing eggs of one or more conveyor rows r1, r2, r3. In a non-limiting example, the light source includes one or more light emitting diodes (LEDs) for emitting the beam B.

Optionally, each light source 2 can be configured to emit or provide a collimated or focused beam B, in particular illuminating a only part of an outer surface of each egg shell of a passing egg (E1, E2). During operation, the light B from the light source 2 can be internally scattered by the eggs E, so that an entire eggs outer contour receives the light (i.e. is illuminated), to be imaged by the camera(s) of the imaging system.

A wavelength of near infrared light (if any) of the illumination beam B, generated by the source 2, can be at least 700 nm and preferably at most 1000 nm, for example a wavelength in the range of 700-800 nm, and preferably a wavelength smaller than 750 nm, in particular a wavelength of about 720 nm. The wavelength of the near infrared light is most preferably in the range of 710-750 nm, preferably the range of 710-730 nm.

In case of emitting white light, the source 2 can be a broadband white light emitter.

In case of emitting blue light, the source 2 can be a broadband or narrowband blue light emitter.

Also, preferably, the system is configured such (e.g. a conveyor transport speed and egg rotation speed is set such that during operation), each egg E1, E2 rotates about a respective longitudinal egg axis over when it is being illuminated by the light beam B.

In the drawing (Fig. 2), the beam source 2 is depicted as emitting the beam towards the detector 3 (see below), but that is not required (in particular in case the eggs internally diffuse received light). The source 2 may operate continuously, but preferably emits the beam intermittently. Also, in an embodiment, operation of the beam source 2 can be synchronized with the conveyor 1 (e.g. with a conveyor speed) such that the source 2 only generates the beam B to illuminate a passing egg, wherein the source does not generate the beam otherwise (e.g. for saving energy and/or to avoid any opposite detector 3 being irradiated directly by the source 2).

Further, the imaging system includes a number of light detectors 3, in particular cameras, arranged for detecting light emanating from the egg conveying path, for example light that is transmitted through (and emitted and/or diffused by) the eggs E1, E2 during operation, and/or light that has not been transmitted through the eggs but has been reflected from the egg surfaces. During operation, the cameras 3 preferably generate a plurality of images of each egg E from different sides of the egg, using illumination light B (that is e.g. transmitted through the egg E).

In this example, the cameras 3 are located at a vertical level above the egg conveyor 1. Thus, contamination of the detectors 3 (e.g. by dirt or other substances that may be present on passing eggs) can be prevented or significantly reduced. Alternatively, one or more (e.g. each) of the cameras 3 can be located at substantially the same vertical level as the passing eggs, and/or for example at a vertical level below the level of the passing eggs. In other words, alternatively, one or more of the cameras 3 can be located at another level, e.g. at or below a level of egg transport, and/or at different locations.

In this example, pairs of cameras 3a, 3b are associated with each one of the transport rows r1, r2, r3 defined by the conveyor 1, and e.g. with one of said light beam sources 2. In particular, for each transport row, at least cameras 3a, 3b are located next to each other when viewed in a top view, for taking egg images from different respective viewing angles. Alternatively, for example, a camera 3 can be installed for detecting light emanating from eggs of several of the rows r1, r2, r3.

Good results can be achieved in case the cameras 3a, 3b are configured to generate grey scale images, to be processed by the image processor 8. Each of the images that are fed to the image processor 8 can e.g. be a bitmap image, for example an 8-bit bitmap image, for example having a resolution of at most 1000x1000 pixels (in particular at most 800x800 pixels). It has been found that such relatively low resolution images can provide good, reliable mass prediction results. Also, according to an embodiment, each camera 3a, 3b can produce a high(er) resolution image, wherein the image processor 8 can be configured to resize and/or crop such a high(er) resolution image to an image of the relatively low(er) resolution of at most 1000x1000 pixels for further processing (via a trained learning model 1001, as will be discussed below).

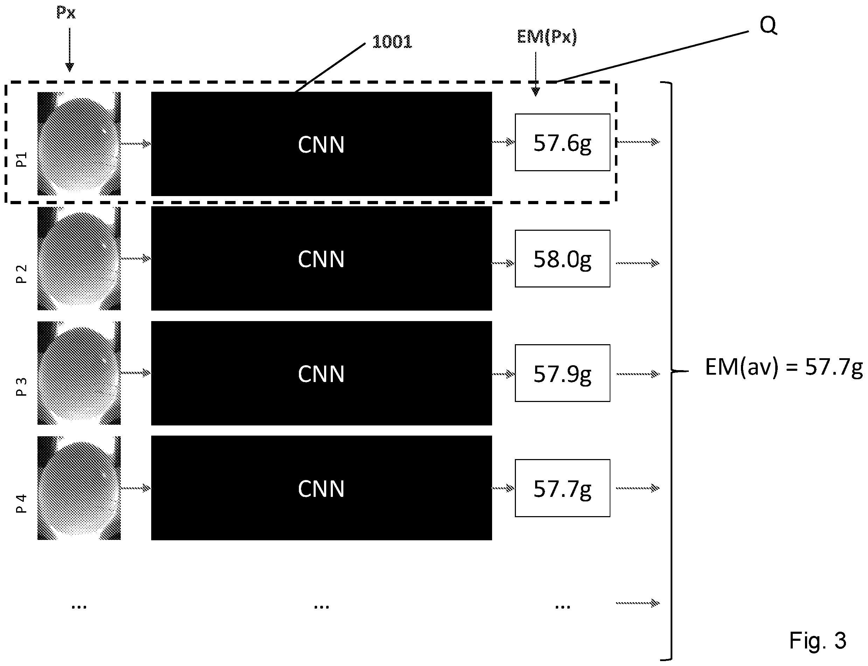

Each camera 3a, 3b can e.g. be configured to generate a detection signal, in particular a digital image of the egg. In a preferred embodiment, each image of each egg (taken by the camera 3a, 3b) encompass the entire illuminated contour of the egg (see Fig. 3).

Optionally, the system can be configured to maintain substantially the same egg illumination conditions/parameters, e.g. when eggs E1, E2 having mutually different eggshell colors are being processed thereby, but that is not required. Therefore, optionally, the same illumination beam B (having the same or constant beam intensity and the same spectrum) can be generated by the source, for illuminating eggs E1, E2. Also, preferably, the respective detector 3 is preferably not adjusted during its operation (independent of whether the detector is detecting light emanating from a first egg or a second egg) to detect light emanating from the plurality of eggs E1, E2 (of the same, respective passing row r1, r2, r3.

Further, the system includes processing means 8 (drawn schematically) configured to process light detection results of the infrared light detector 3. The processing means 8 can be configured in various ways, and can include e.g. processor software, processor hardware, a computer, data processing means, a memory for storing data to be processed, et cetera. Also, the processor means 8 can include or be connected to various respective communication means for allowing communication with the light detectors 3 for receiving detection results thereof, or the processing means 8 and detector(s) 3 can be integrated with each other. Besides, the processing means 8 can include e.g. a user interface for allowing operator interaction with the central processor, and e.g. for outputting data processed by the processor.

In particular, the processing means is a digital image processor 8, configured to receive the plurality N of images from the imaging system 2, 3, and to process the plurality of images of each egg E utilizing a trained learning model 1001 to estimate a mass of that egg. Herein, it is preferred that the imaging system 2, 3 is configured to take at least 4 images (N=4) of each passing egg and preferably at least 10 or 20 images, for example 25 images or more, to be processed by the image processor 8 (via the trained learning model 1001).

Also, the image processor 8 is configured to output a determined mass ME of the egg, for example for generating and/or storing the determined mass ME in association with a predetermined egg identifier (e.g. a unique code or number) of the respective egg and/or for classifying the eggs. The output can e.g. be stored in a memory and/or server, and/or provided to an operator via a user interface or the like, as will be clear to the skilled person.

As follows from the above, the conveyor 1 is configured to rotate the eggs E during transport along the conveying path, in particular when the eggs pass along the imaging system 2, 3. In this way, the imaging system 2, 3 can generate a plurality of images of each egg E from a different viewing angles, i.e., from mutually different directions (with respect to the egg's outer surface). It is preferred that the conveyor 1 and the imaging system 2, 3 are configured to cooperate (e.g. by the imaging system taking the plurality N of images during a predetermined egg rotation as provided by the conveyor 1) such that the respective plurality of images of an egg E contains an entire outer surface (i.e. all sides) of the egg E. The two (or more) camera's 3a, 3b associated with one of the conveying paths r1, r2, r3 can e.g. take respective egg images at the same time. Also, time periods between the taking of two subsequent images of each egg E by each camera 3a, 3b can be constant (in particular in case of a constant egg rotation speed as provided by the conveyor 1).

Preferably, the learning model 1001 has been trained through machine learning with training data including a plurality of images of a plurality of eggs and determined (actual) masses MM of those eggs. The determined (actual) egg masses MM, used for the training of the model, can e.g. be determined using egg weighing devices (e.g. scales), known as such, e.g. manually or in an automated egg weighing system.

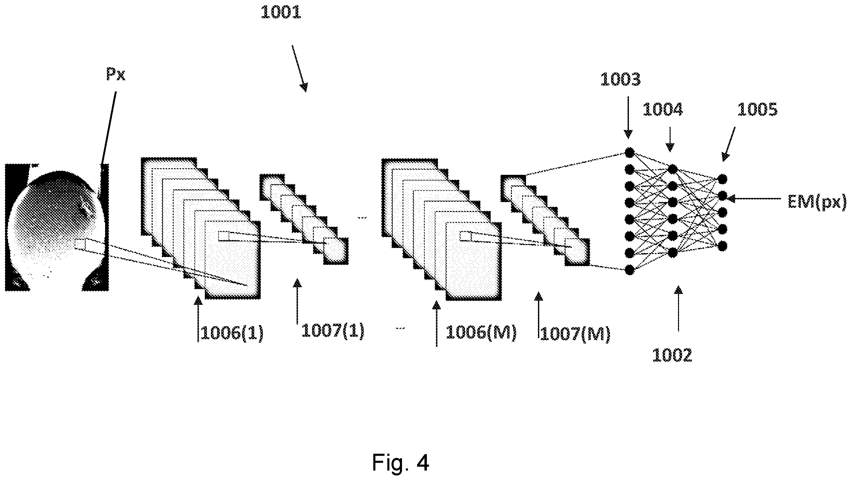

The trained learning model 1001 preferably includes a neural network, preferably a CNN (known as such, see e.g. https:/en.wikipedia.org/wiki/Convolutional_neural_network). An example of the model 1001 is depicted in Figures 3, 4 and will be explained below in more detail.

For example, an initial operation of the system can involve a method of training the machine learning model 1001 to predict the mass of an avian egg.

The process of learning the model 1001 (e.g. a CNN) can be a process of finding the values of all learning model parameters within a learning model network, such that an input image (of the egg) results in result values with a high correlation to the actual weight of the act (that is to be measured).

The training method can including at least the following steps:
providing the machine learning model 1001, which is adapted to receive input data and to output prediction data, wherein the training includes:
- A) the conveyor 1 conveying a plurality of eggs E along a conveying path r1, r2, r3;
- B) the imaging system 2, 3 generating a set (i.e. a plurality N) of training images of each of the eggs E (being conveyed and e.g. rotated by the conveyor 1) from different sides of the egg, using the illumination light B;
- C) measuring the (actual) mass MM of each of the eggs E; and
- D) feeding the plurality of training images of each of the eggs E and the measured mass MM of that egg E to the machine learning model to train the learning model to output egg mass ME as prediction data.

Herein, it is preferred that a relatively large number of eggs is processed for the training, wherein preferably training images and corresponding measured masses of at least 10,000 different eggs are fed to the machine learning model to train the learning model. Also, as is mentioned before, a set (plurality N) of training images of each egg that is fed to the learning model 1001 preferably includes at least 4 images of that egg and preferably at least 10 or 20 images, for example 25 images or more, for example in the range of 20-30 images.

After the learning model 1001 has been trained, the respective system can carry out a method for processing eggs E. The method includes:
- conveying each egg E along a conveying path r1, r2, r3;
- generating a plurality of images of each egg E (being conveyed, and preferably rotated, by the conveyor) from different sides of the egg using illumination light B (that is for example transmitted through the egg E); and
processing the plurality of images of each egg E, by a digital image processor 8, utilizing the trained learning model to determine a mass ME of the egg, and outputting the determined mass ME of the egg, for example for classifying the egg.

As follows above, again, during system egg processing operation, the plurality of images of each egg E encompass the entire outer surface of the egg. Each egg E is preferably rotated during a sequence of generating the plurality N of images of the egg E. Preferably, the number N of images of each egg during the training of the learning model 1001 is the same as the number N of images of each egg during subsequent normal system operation when using the trained learning model.

Also, the trained learning model 1001 can use substantially raw image data provided by the at least one camera 3. Herein, raw image data in particular means an image as produced by the camera 3 without significant image processing applied thereto before the image is being fed to the machine learning model. Some image processing can be applied, for example a cropping operation for cropping to a section of an image that contains the entire contour of the imaged egg, wherein another section of that images that does not contain the egg is removed, before the (cropped) images is fed to the machine learning model. Also, image processing can include changing a size or lowering a resolution of the image, before the image is fed to the trained learning model 1001. The raw image data of each (optionally cropped or resized) image is preferably a low resolution grey scale image, for example having at most 1000x1000 pixels (e.g. less than 900x900 pixels).

Figures 3 and 4 depict an example of a learning model architecture and corresponding steps that can be carried out by the learning model 1001, in particular a CNN (e.g. executed by the system's image processor 8).

Figure 3 depicts a number of pictures Px (P1, P2, P3, P4, ...., P_{N}) of a single egg, taken from different viewing angles by the imaging system 2, 3. In total, a plurality N of images is taken of each egg E. The N images are preferably greyscale bitmap images (as mentioned above), preferably of low resolution. During operation, each of the N images Px is fed into the learning model 1001 (executed by the image processor 8), to be processed thereby, for estimating an egg mass EM(Px) associated with that image Px. After all egg masses EM(Px) concerning all N images of the egg E have been determined, the image processor 8 can calculate a mean egg mass EM (i.e. EM equals the sum of the N determined egg masses EM(Px) divided by N), and output that mass EM as the estimated egg mass of the egg E.

Figure 4 shows an example of the above-mentioned learning model 1001, in particular processing one of the plurality egg images Px of the egg (to estimate a respective egg mas EM(Px)). The learning model 1001 preferably includes a number of fully connected layers 1003, 1004, 1005 (i.e. layers where each input pixel is multiplied by a parameter value), for example a neural network 1002 (see Figure 4). The fully connected layers include an input layer 1003, at least one intermediate layer 1004, and an output layer 1005 each having multiple nodes. For example, the input layer can have multiple nodes 1003 to which data from a pooling layer 1006 (see below) is input. The intermediate layer can have multiple nodes 1004 each performing arithmetic on the data input from each of the nodes 1003 of the input layer by using parameters. The multiple nodes 1004 respectively output data to multiple nodes 1005 of the output layer. The nodes 1005 of the output layer include at least include a node for outputting the determined (estimated) egg mass EM(Px). In an embodiment, only a single output node 1005 is present for outputting the determined egg mass EM(Px).

The pooling layer 1006 of the CNN is part of a set of layers including M convolutional layers 1006(1,..,M) and M associated pooling layers 1007(1, ...., M). Each convolution layer 1006(1,....,M) is configured to apply a filter matrix across the image (convolve), received from its previous layer. Each pooling layer is configured to reduce the amount of pixels (of the image received from a previous convolution layer) by taking a max value of the image area. The first of the convolution layers 1006(1) receives the image Px as generated by the imaging system 2, 3.

In particular, during operation, each convolutional layer 1006 can convolve its inputs (pixels) with a filter matrix of a certain dimension. Each value in the matrix is a parameter which can be "tuned" by the learning algorithm (during the training of the model). For example, as is common knowledge, in case a gray level image of size 512x512x1 (1 is the number of channels) is used as input, a 3x3 kernel with 32 filters and bias results in 1 * 3 * 3 * 32 + 32 = 320 parameters. Without bias this results in 1 * 3 * 3 * 32 = 288. A

According to an embodiment, all layers (including the convolutional layers and the fully connected layers) combined of the learning model 1001 have a total of less than 2 million parameters, for example only 1.6 million parameters. Thus, the processing of the egg images by the image processor 8 can be carried out real-time in case a relatively inexpensive digital image processor 8 is implemented.

It has been found that in this way, egg mass can be estimated efficiently, without e.g. requiring complex 3D rendering techniques for determining masses of individual egg components (e.g. egg shell, egg liquid). Moreover, a relatively simple learning algorithm can be used, so that it can be executed by relatively low-cost image processor hardware.

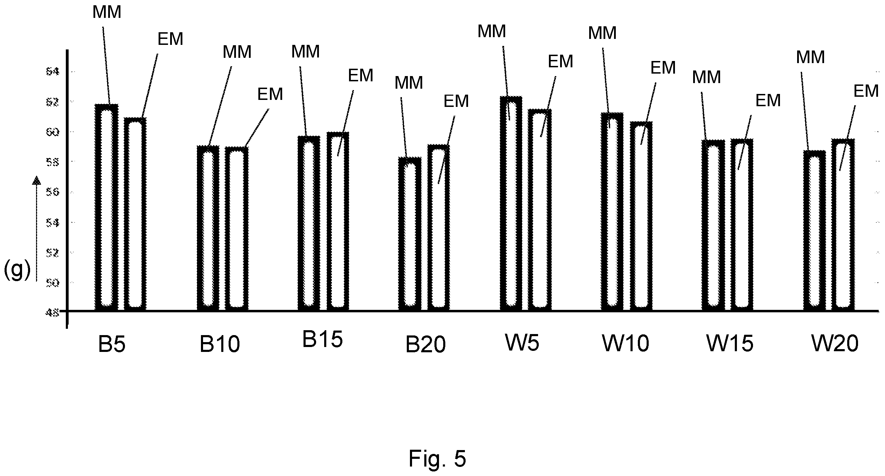

Figure 5 shows results of the present system and method, indicating estimated masses EM of several types of eggs (as estimated by the present system) compared to actual, measured masses MM. The different types of eggs include several types of brown eggs B5, B10, B15, B20 and several types of white eggs W5, W10, W15, W20, wherein differences between the eggs concern egg storage times (egg age). It follows that the estimated masses EM match the actual (measured) masses MM surprisingly well.

Without wishing to be bound to any theory it is believed that the present system and method can process relatively simple grey scale bitmap egg images that show the entire shape of the egg as well as potential air chamber related features within the egg, respective dimensions, possibly in combination with egg shell information. The learning model automatically takes such egg related features into account during the learning process, after which the learned learning model can provide good egg mass predictions.

It is self-evident that the invention is not limited to the above-described exemplary embodiments. Various modifications are possible within the framework of the invention as set forth in the appended claims.

In this application, the eggs to be processed are in particular nonliving, dead eggs, i.e. eggs for consumption that are not fertilized (and do not contain any embryo). The avian/bird eggs can be poultry eggs, for example chicken eggs.

Also, in a preferred embodiment, the detector, detecting (transmitted) near infrared light, maintains a certain predetermined light detecting state during detecting light resulting from eggs having mutually different shell color, without hampering detecting results (and subsequent processing results). However, that is not essential. Alternatively, for example, at least part of the detector may change a respective state with respect of detecting light emanating from various (e.g. different) eggs.

Also, it will be clear that each camera can be located at various locations, and can be configured e.g. to detect light that has been transmitted from a single egg or light that has been transmitted from a plurality of eggs. Besides, alternatively, at least one or each camera can be configured to detect illumination light that has not been transmitted through the egg, but has only illuminated an outer surface of the egg, for example light that has been reflected by the egg. Similarly, at least one or each camera can be configured to detect both transmitted illumination light (that has been transmitted through the egg) and non-transmitted illumination light (i.e. light that has not passed the egg).

For example, the system can include a plurality of camera for viewing an egg from different viewing directions. For example, at least one camera-type detector can be provided, each camera being arranged for taking images of one or more eggs, to be examined, at the same time. In that case, e.g. an image taken by a camera can be divided or cropped into several images, associated with different eggs, to be fed to the image processor as egg images.

Furthermore, each said light source can be arranged at various positions, for example below, at and/or above a vertical level of the egg(s). Moreover, a plurality of light sources can be implemented for illuminating (e.g. simultaneously) illuminating a single egg E1, E2, from the same or from different directions.

Also, for example, the color of the eggshell can be the color as perceived by the naked (human) eye as will be appreciated by the skilled person.

Besides, the methods according to the above-described example embodiments may be embodied by the digital image processor executing instructions stored in non-transitory computer-readable media including program instructions. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

Software may include a computer program, a piece of code, an instruction, or some combination thereof, to independently or collectively instruct or configure the digital image processor to operate as desired. Software and data may be embodied permanently or temporarily in any type of machine, component, physical or virtual equipment, computer storage medium or device, or in a propagated signal wave capable of providing instructions or data to or being interpreted by image processor. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

While this disclosure includes specific example embodiments, it will be apparent to one of ordinary skill in the art that various changes in form and details may be made in these example embodiments without departing from the scope of the claims.

For example, it is preferred that a large number N of images is used for each egg, wherein the images can be sequentially taken from two different angles during egg rotation, but also images from a single angle (i.e. taken a by a single camera) or images from three or more angles can be used.

Also, the method can include using a standard convolutional neural network 1001, for example in combination with Bayesian optimization for hyperparameter search.

Besides, illumination light of various wavelengths or wavelength ranges can be used. Optionally, NIR light is used as illumination light. Alternatively or additionally, white light (e.g. having wavelengths ranging from about 400 to 700 nm) is used, and/or blue light (having one wavelength or a range of wavelengths in the range of about 380 to 500 nm).

Also, it will be appreciated that the learning model 1001 can include a number of fully connected layers 1003, 1004, 1005 (see e.g. Fig. 4), and also a number of ReLU (rectified linear unit) activation layers (which can be part of all fully connected blocks, and part of all convolutional blocks; herein, a block can be defined as a set of combined layers, for instance: Fully connected Block: FullyConnected -> ReLU. Convolutional Block: Convolution -> BatchNorm -> ReLU -> AveragePool2D).

## Claims

1. Method for processing a plurality of avian eggs, in particular unfertilized eggs, including:
- conveying each egg (E) along a conveying path;
- generating a plurality of images of each egg (E) from different sides of the egg using illumination light (B); and
processing the plurality of images of each egg (E), by a digital image processor (8), utilizing a trained learning model to determine a mass of the egg, and outputting the determined mass of the egg, for example for classifying the egg.

2. The method according to claim 1, wherein the learning model has been trained through machine learning with training data concerning a plurality of eggs, the training data including a plurality of images of each of the plurality of eggs and determined masses of the eggs.

3. The method according to any of the preceding claims, wherein the trained learning model includes a neural network, preferably a CNN.

4. The method according to any of the preceding claims, wherein the plurality of images of each egg (E) encompass the entire outer surface of the egg.

5. The method according to any of the preceding claims, wherein each egg (E) is rotated during the generating of the plurality of images of the egg.

6. The method according to any of the preceding claims, wherein the plurality of images include at least 4 images of the egg and preferably at least 10 or at least 20 images, for example 25 images or more, for example in the range of 20-30 images.

7. Method according to any of the preceding claims, wherein each of the images is a grey scale image.

8. Method according to any of the preceding claims, wherein each of the images is a bitmap image, for example an 8-bit bitmap image, for example having a resolution of at most 1000x1000 pixels.

9. Method according to any of the preceding claims, wherein the plurality of images of each egg is generated by at least one camera (3), wherein the trained learning model uses substantially raw image data provided by the at least one camera (3).

10. Method according to any of the preceding claims, wherein infrared light (B), preferably near infrared light, is used for imaging the eggs, wherein a wavelength of the light (B) is preferably smaller than 800 nm, for example smaller than 750 nm, wherein the wavelength of the light (B) is for example in the range of 710-750 nm, preferably the range of 710-730 nm.

11. Method according to any of the preceding claims, wherein white light (B) is used for imaging the eggs.

12. Method according to any of the preceding claims, wherein blue light (B) is used for imaging the eggs.

13. System for processing a plurality of avian eggs, in particular unfertilized eggs, the system including:
- a conveyor (1) for conveying each egg (E) along a conveying path;
- an imaging system having at least one camera (3) and at least one light source (2), for generating a plurality of images of each egg (E) conveyed by the conveyor (1) from different sides of the egg, wherein the at least one camera (3) and the at least one light source (2) are arranged on opposite sides of the conveying path;
- a digital image processor (8) configured to receive the plurality of images from the imaging system, and to process the plurality of images of each egg (E) utilizing a trained learning model to determine a mass of the egg,
wherein the image processor is configured to output a determined mass of the egg, for example for generating and/or storing the determined mass in association with a predetermined egg identifier of the respective egg and/or for classifying the eggs.

14. System according to claim 13, wherein the conveyor is configured to rotate the eggs during transport along the conveying path, in particular when the eggs pass along the imaging system (2, 3).

15. System according to claim 13 or 14, wherein the imaging system includes at least two cameras (3a, 3b), arranged to take images of eggs (E) passing along the conveying path from mutually different directions.

16. System according to any of claims 13-15, wherein the conveyor (1) and the imaging system (2, 3) are configured to cooperate such that the respective plurality of images of an egg (E) contains an entire outer surface of the egg (E).

17. System according to any of claims 13-16, wherein the imaging system is configured to take at least 4 images of a passing egg and preferably at least 10 or at least 20 images, for example 25 images or more, to be processed by the image processor (8).

18. System according to any of claims 13-17, wherein the learning model has been trained through machine learning with training data including a plurality of images of a plurality of eggs and determined masses of those eggs, wherein the trained learning model preferably includes a neural network, preferably a CNN.

19. A digital image processor comprising means for carrying out the processing of the plurality of images of each egg (E), utilizing the trained learning model to determine a mass of the egg, of the method according to any one of claims 1-12.

20. Method of training a machine learning model to predict the mass of an avian egg, including:
providing a machine learning model, which is adapted to receive input data and to output prediction data, wherein the training includes:
- A) a conveyor (1) conveying a plurality of eggs (E) along a conveying path;
- B) an imaging system (2, 3) generating a plurality of training images of each of the eggs (E) from different sides of the egg, using illumination light (B);
- C) measuring the mass of each of the eggs; and
- D) feeding the plurality of training images of each of the eggs and the measured mass of the egg to the machine learning model to train the learning model to output egg mass as prediction data,
wherein preferably training images and corresponding measured masses of at least 10,000 different eggs are fed to the machine learning model to train the learning model.

21. Method according to claim 20, wherein the plurality of training images of each egg includes at least 4 images of the egg and preferably at least 10 or at least 20 images, for example 25 images or more, for example in the range of 20-30 images.

22. Method according to any of claims 20-21, wherein the trained learning model includes a neural network, preferably a CNN.

## Patentansprüche

1. Verfahren zur Verarbeitung einer Vielzahl von Vogeleiern, insbesondere unbefruchteter Eier, einschließlich:
- Befördern jedes Eies (E) entlang eines Förderweges;
- Erzeugen einer Vielzahl von Bildern von jedem Ei (E) von verschiedenen Seiten des Eies unter Verwendung von Beleuchtungslicht (B); und
Verarbeiten der Vielzahl von Bildern jedes Eies (E) durch einen digitalen Bildprozessor (8) unter Verwendung eines trainierten Lernmodells, um eine Masse des Eies zu bestimmen, und Ausgeben der bestimmten Masse des Eies, zum Beispiel zum Klassifizieren des Eies.

2. Verfahren nach Anspruch 1, wobei das Lernmodell durch maschinelles Lernen mit Trainingsdaten trainiert wurde, die Vielzahl von Eiern betreffen, wobei die Trainingsdaten eine Vielzahl von Bildern von jedem der Vielzahl von Eiern und bestimmte Massen der Eier einschließen.

3. Verfahren nach einem der vorstehenden Ansprüche, wobei das trainierte Lernmodell ein neuronales Netz, vorzugsweise ein CNN, einschließt.

4. Verfahren nach einem der vorstehenden Ansprüche, wobei die einen Vielzahl von Bildern eines jeden Eies (E) die gesamte Außenfläche des Eies beinhalten.

5. Verfahren nach einem der vorstehenden Ansprüche, wobei jedes Ei (E) während des Erzeugens der Vielzahl von Bildern des Eies gedreht wird.

6. Verfahren nach einem der vorstehenden Ansprüche, wobei die Vielzahl von Bildern mindestens 4 Bilder des Eies und vorzugsweise mindestens 10 oder mindestens 20 Bilder, zum Beispiel 25 Bilder oder mehr, zum Beispiel in dem Bereich von 20 bis 30 Bildern, einschließen.

7. Verfahren nach einem der vorstehenden Ansprüche, wobei jedes der Bilder ein Graustufenbild ist.

8. Verfahren nach einem der vorstehenden Ansprüche, wobei jedes der Bilder ein Bitmap-Bild ist, z. B. ein 8-Bit-Bitmap-Bild, z. B. mit einer Auflösung von höchstens 1000x1000 Pixeln.

9. Verfahren nach einem der vorstehenden Ansprüche, wobei die Vielzahl von Bildern jedes Eies von mindestens einer Kamera (3) erzeugt werden, wobei das trainierte Lernmodell im Wesentlichen Rohbilddaten verwendet, die von der mindestens einen Kamera (3) bereitgestellt werden.

10. Verfahren nach einem der vorstehenden Ansprüche, wobei Infrarotlicht (B), vorzugsweise Nahinfrarotlicht, zum Abbilden der Eier verwendet wird, wobei eine Wellenlänge des Lichts (B) vorzugsweise kleiner als 800 nm, beispielsweise kleiner als 750 nm ist, wobei die Wellenlänge des Lichts (B) beispielsweise in dem Bereich von 710 bis 750 nm, vorzugsweise in dem Bereich von 710 bis 730 nm liegt.

11. Verfahren nach einem der vorstehenden Ansprüche, wobei weißes Licht (B) zum Abbilden der Eier verwendet wird.

12. Verfahren nach einem der vorstehenden Ansprüche, wobei blaues Licht (B) zum Abbilden der Eier verwendet wird.

13. System zur Verarbeitung einer Vielzahl von Vogeleiern, insbesondere unbefruchteter Eier, wobei das System Folgendes einschließt:
- einem Förderer (1) zum Befördern jedes Eies (E) entlang eines Förderweges;
- ein Abbildungssystem mit mindestens einer Kamera (3) und mindestens einer Lichtquelle (2) zum Erzeugen einer Vielzahl von Bildern von jedem Ei (E), das von dem Förderer (1) befördert wird, und zwar von verschiedenen Seiten des Eies, wobei die mindestens eine Kamera (3) und die mindestens eine Lichtquelle (2) auf gegenüberliegenden Seiten des Förderweges angeordnet sind;
- einen digitalen Bildprozessor (8), der konfiguriert ist, um die Vielzahl von Bilder von dem Abbildungssystem zu empfangen und die Vielzahl von Bilder jedes Eies (E) unter Verwendung eines trainierten Lernmodells zu verarbeiten, um eine Masse des Eies zu bestimmen, wobei der Bildprozessor konfiguriert ist, um eine bestimmte Masse des Eies auszugeben, beispielsweise zum Erzeugen und/oder Speichern der bestimmten Masse in Verbindung mit einem vorbestimmten Eieridentifikator des jeweiligen Eies und/oder zum Klassifizieren der Eier.

14. System nach Anspruch 13, wobei der Förderer konfiguriert ist, um die Eier während des Transports entlang des Förderweges zu drehen, insbesondere wenn die Eier an dem Abbildungssystem (2, 3) vorbeilaufen.

15. System nach Anspruch 13 oder 14, wobei das Abbildungssystem mindestens zwei Kameras (3a, 3b) einschließt, die angeordnet sind, um Bilder von Eiern (E), die entlang des Förderweges vorbeilaufen, aus zueinander unterschiedlichen Richtungen aufzunehmen.

16. System nach einem der Ansprüche 13 bis 15, wobei der Förderer (1) und das Abbildungssystem (2, 3) konfiguriert sind, um derart zusammenzuwirken, dass die jeweiligen eine Vielzahl von Bildern eines Eies (E) eine gesamte Außenfläche des Eies (E) enthalten.

17. System nach einem der Ansprüche 13 bis 16, wobei das Abbildungssystem konfiguriert ist, um mindestens 4 Bilder eines vorbeilaufenden Eies und vorzugsweise mindestens 10 oder mindestens 20 Bilder, zum Beispiel 25 Bilder oder mehr, aufzunehmen, die vom Bildprozessor (8) verarbeitet werden.

18. System nach einem der Ansprüche 13 bis 17, wobei das Lernmodell durch maschinelles Lernen mit Trainingsdaten trainiert wurde, die mehrere Bilder einer Mehrzahl von Eiern und bestimmte Massen dieser Eier einschließen, wobei das trainierte Lernmodell vorzugsweise ein neuronales Netz, vorzugsweise ein CNN, einschließt.

19. Digitaler Bildprozessor, umfassend Mittel zum Ausführen der Verarbeitung der Vielzahl von Bildern jedes Eies (E) unter Verwendung des trainierten Lernmodells, um eine Masse des Eies zu bestimmen, und zwar gemäß dem Verfahren nach einem der Ansprüche 1 bis 12.

20. Verfahren zum Trainieren eines maschinellen Lernmodells, um die Masse eines Vogeleis vorherzusagen, das Folgendes einschließt:
Bereitstellen eines maschinellen Lernmodells, das geeignet ist,
Eingangsdaten zu empfangen und Vorhersagedaten auszugeben, wobei das Training einschließt:
- A) einen Förderer (1), einer Vielzahl von Eiern (E) entlang eines Förderweges befördert;
- B) ein Abbildungssystem (2, 3), das unter Verwendung von Beleuchtungslicht (B) eine Vielzahl von Trainingsbilder von jedem der Eier (E) von verschiedenen Seiten des Eies erzeugt;
- C) Messen der Masse jedes einzelnen Eies; und
- D) Einspeisen der Vielzahl von Trainingsbilder von jedem der Eier und der gemessenen Masse des Eies in das maschinelle Lernmodell, um das Lernmodell so zu trainieren, dass es die Eimasse als Vorhersagedaten ausgibt,
wobei vorzugsweise Trainingsbilder und entsprechende gemessene Massen von mindestens 10.000 verschiedenen Eiern in das maschinelle Lernmodell eingespeist werden, um das Lernmodell zu trainieren.

21. Verfahren nach Anspruch 20, wobei die einem Vielzahl von Trainingsbilder jedes Eies mindestens 4 Bilder des Eies und vorzugsweise mindestens 10 oder mindestens 20 Bilder, zum Beispiel 25 Bilder oder mehr, zum Beispiel in dem Bereich von 20 bis 30 Bildern, einschließen.

22. Verfahren nach einem der Ansprüche 20 bis 21, wobei das trainierte Lernmodell ein neuronales Netz, vorzugsweise ein CNN, einschließt.

## Revendications

1. Procédé de traitement d'une pluralité d'œufs aviaires, en particulier des œufs non fertilisés, incluant :
de convoyer chaque œuf (E) le long d'un chemin de convoyage ;
de générer une pluralité d'images de chaque œuf (E) depuis différents côtés de l'œuf en utilisant une lumière d'éclairage (B) ; et
de traiter la pluralité d'images de chaque œuf (E), par un dispositif de traitement d'image numérique (8), en utilisant un modèle d'apprentissage entraîné pour déterminer une masse de l'œuf, et de sortir la masse déterminée de l'œuf, par exemple pour classifier l'œuf.

2. Procédé selon la revendication 1, dans lequel le modèle d'apprentissage a été entraîné par apprentissage automatique avec des données d'entraînement concernant une pluralité d'œufs, les données d'entraînement incluant une pluralité d'images de chacun de la pluralité d'œufs et les masses déterminées des œufs.

3. Procédé selon l'une quelconque des revendications précédentes, dans lequel le modèle d'apprentissage entraîné inclut un réseau neuronal, de préférence un CNN.

4. Procédé selon l'une quelconque des revendications précédentes, dans lequel la pluralité d'images de chaque œuf (E) couvre toute la surface extérieure de l'œuf.

5. Procédé selon l'une quelconque des revendications précédentes, dans lequel chaque œuf (E) est tourné pendant la génération de la pluralité d'images de l'œuf.

6. Procédé selon l'une quelconque des revendications précédentes, dans lequel la pluralité d'images inclut au moins quatre images de l'œuf et de préférence au moins 10 ou au moins 20 images, par exemple 25 images ou plus, par exemple dans la plage de 20-30 images.

7. Procédé selon l'une quelconque des revendications précédentes, dans lequel chacune des images est une image en niveaux de gris.

8. Procédé selon l'une quelconque des revendications précédentes, dans lequel chacune des images est une image bitmap, par exemple une image bitmap 8 bits, par exemple ayant une résolution d'au plus 1000x1000 pixels.

9. Procédé selon l'une quelconque des revendications précédentes, dans lequel la pluralité d'images de chaque œuf est générée par au moins une caméra (3), dans lequel le modèle d'apprentissage entraîné utilise sensiblement des données d'image brute fournies par l'au moins une caméra (3).

10. Procédé selon l'une quelconque des revendications précédentes, dans lequel de la lumière infrarouge (B), de préférence de la lumière infrarouge proche, est utilisée pour imager les œufs, dans lequel une longueur d'onde de la lumière (B) est de préférence inférieure à 800 nm, par exemple inférieure à 750 nm, dans lequel la longueur d'onde de la lumière (B) est par exemple dans la plage de 710-750 nm, de préférence la plage de 710-730 nm.

11. Procédé selon l'une quelconque des revendications précédentes, dans lequel de la lumière blanche (B) est utilisée pour imager les œufs.

12. Procédé selon l'une quelconque des revendications précédentes, dans lequel de la lumière bleue (B) est utilisée pour imager les œufs.

13. Système pour traiter une pluralité d'œufs aviaires, en particulier des œufs non fertilisés, le système incluant :
un convoyeur (1) pour convoyer chaque œuf (E) le long d'un chemin de convoyage ;
un système d'imagerie comportant au moins une caméra (3) et au moins une source de lumière (2), pour générer une pluralité d'images de chaque œuf (E) convoyé par le convoyeur (1) depuis différents côtés de l'œuf, dans lequel l'au moins une caméra (3) et l'au moins une source de lumière (2) sont agencées sur des côtés opposés du chemin de convoyage ;
un dispositif de traitement d'image numérique (8) configuré pour recevoir la pluralité d'images depuis le système d'imagerie, et pour traiter la pluralité d'images de chaque œuf (E) en utilisant un modèle d'apprentissage entraîné pour déterminer une masse de l'œuf, dans lequel le dispositif de traitement d'image est configuré pour sortir une masse déterminée de l'œuf, par exemple pour générer et/ou stocker la masse déterminée en association avec un identificateur d'œuf prédéterminé de l'œuf respectif et/ou pour classifier les œufs.

14. Système selon la revendication 13, dans lequel le convoyeur est configuré pour faire tourner les œufs pendant le transport le long du chemin de convoyage, en particulier quand les œufs passent le long du système d'imagerie (2, 3).

15. Système selon la revendication 13 ou 14, dans lequel le système d'imagerie inclut au moins deux caméras (3a, 3b) agencées pour prendre des images des œufs (E) passant le long du chemin de convoyage depuis des directions mutuellement différentes.

16. Système selon l'une quelconque des revendications 13-15, dans lequel le convoyeur (1) et le système d'imagerie (2, 3) sont configurés pour coopérer de telle manière que la pluralité d'images respectives d'un œuf (E) contient une surface extérieure entière de l'œuf (E).

17. Système selon l'une quelconque des revendications 13-16, dans lequel le système d'imagerie est configuré pour prendre au moins quatre images d'un œuf passant et de préférence au moins 10 ou au moins 20 images, par exemple 25 images ou plus, pour être traitées par le dispositif de traitement d'image (8).

18. Système selon l'une quelconque des revendications 13-17, dans lequel le modèle d'apprentissage a été entrainé par apprentissage automatique avec des données d'entraînement incluant une pluralité d'images d'une pluralité d'œufs et des masses déterminées de ces œufs, dans lequel le modèle d'apprentissage entraîné inclut de préférence un réseau neuronal, de préférence un CNN.

19. Dispositif de traitement d'image numérique comprenant des moyens pour réaliser le traitement de la pluralité d'images de chaque œuf (E), en utilisant le modèle d'apprentissage entraîné pour déterminer une masse de l'œuf, du procédé selon l'une quelconque des revendications 1-12.

20. Procédé d'entraînement d'un modèle d'apprentissage automatique pour prédire la masse d'un œuf aviaire, incluant ;
de fournir un modèle d'apprentissage automatique, qui est adapté pour recevoir des données d'entrée et pour sortir des données de prédiction, dans lequel l'entraînement inclut :
A) un convoyeur (1) convoyant une pluralité d'œuf (E) le long d'un chemin de convoyage ;
B) un système d'imagerie (2, 3) générant une pluralité d'images d'entraînement de chacun des œufs (E) convoyés par le convoyeur (1) depuis différents côtés de l'œuf, en utilisant la lumière d'éclairage (B) ;
C) mesurer la masse de chacun des œufs ; et
D) alimenter la pluralité d'images d'entraînement de chacun des œufs et la masse mesurée de l'œuf dans le modèle d'apprentissage entraîné pour entraîner le modèle d'apprentissage pour sortir une masse d'œuf comme donnée de prédiction,
dans lequel de préférence des images d'entraînement et des masses mesurées correspondantes d'au moins 10000 œufs différents sont alimentées vers le modèle d'apprentissage automatique pour entraîner le modèle d'apprentissage.

21. Procédé selon la revendication 20, dans lequel la pluralité d'images d'entraînement de chaque œuf inclut au moins 4 images de l'œuf et de préférence au moins 10 ou au moins 20 images, par exemple 25 images ou plus, par exemple dans la plage de 20-30 images.

22. Procédé selon l'une quelconque des revendications 20-21, dans lequel le modèle d'apprentissage entraîné inclut un réseau neuronal, de préférence un CNN.
